# EUROPEAN PATENT SPECIFICATION

(11) **EP 0 644 746 B1**
(45) Date of publication and mention of the grant of the patent: **15.09.1999**
(21) Application number: 93913793.1
(22) Date of filing: 03.05.1993
(51) Int. Cl.: A61F 13/00

(54) **THE USE OF GLYCERIN IN MODERATING TRANSDERMAL DRUG DELIVERY**
VERWENDUNG VON GLYZERIN ZUR DÄMPFUNG DER TRANSDERMALEN ARZNEIMITTELVERABREICHUNG
UTILISATION DE GLYCERINE POUR MODERER UN APPORT MEDICAMENTEUX TRANSDERMIQUE

(30) Priority: 11.06.1992 US 897269
(43) Date of publication of application: 29.03.1995
(73) Proprietor: THERATECH, INC., Salt Lake City Utah 84108 (US)
(72) Inventor: HEIBER, Werner, E., Salt Lake City, UT 84108 (US); EBERT, Charles, D., Salt Lake City, UT 84108 (US); PATEL, Dinesh, C., Murray, UT 84107 (US); VENKATESHWARAN, Srinivasan, Salt Lake City, UT 84108 (US)
(74) Representative: Thomson, Paul Anthony
(86) International application number: US9304152
(87) International publication number: WO9325168

(56) References cited:
- EP-A- 0 020 905
- EP-A- 0 469 745
- US-A- 4 855 294
- US-A- 4 879 274
- US-A- 5 059 189
- CHEM. ABSTRACTS, Vol. 112, 204738N Topical Pharmaceutical and Cosmetic Gel Base Containing Oil-In-Water Emulsion (KOBAYASHI) 1990, see entire document.
- CHEM. ABSTRACTS, Vol. 112, 185670R Medicament Release from Ointment Bases v. Naproxen In Vitro Release and In Vivo Percutaneous Absorption in Rabbits (RAHMAN) 1990, see entire document.
- CHEM. ABSTRACTS, Vol. 110, 219086F Sustained - Release Topical Analgesics Containing Hydrogels, Polymers and Solubilizers (KITA) 1988, see entire document.

## Description

### FIELD OF INVENTION

The invention relates generally to a method for moderating, altering or controlling the delivery of agents across biological membranes including skin or mucosa. More particularly the invention relates to the use of glycerin to reduce initial high delivery rates while maintaining a more uniform drug penetration over the intended duration of application of a transdermal or transmucosal drug delivery system. The invention thus encompasses a method of moderating drug penetration by co-administering glycerin with various transdermal/transmucosal delivery compositions which may or may not contain permeation enhancers.

### BACKGROUND OF THE INVENTION

The oral administration of drugs as currently employed is undesirable for a number of reasons. First, drugs with short half lives require frequent dosing (2 to 4 times daily) which can lead to inadequate compliance. Second, the short plasma half life of the drug and frequent dosing regimen result in "peaks" and "valleys" in the plasma concentration profile, which increases the likelihood of adverse side effects associated with the peak concentration as well as lapse on therapeutic effectiveness towards the end of the dosing interval. Third, the potential hepatic first pass metabolism associated with oral administration could lead to poor bioavailibility of the drug. Thus, an effective and consistent transdermal drug delivery system for such drugs would be far superior to the current oral regimen.

Transdermal delivery of drugs provides many advantages over conventional oral administration. Advantages of transdermal systems include convenience, noninterrupted therapy, improved patient compliance, reversibility of treatment (by removal of the system from the skin), elimination of "hepatic first pass" effect, the high degree of control over blood concentration of any particular drug and consequent reduction of side effects.

Although transdermal systems have many advantages, most drugs are not amenable to this mode of administration due to the well known barrier properties of the skin. Molecules moving from the environment into and through intact skin must first penetrate the stratum corneum and any material on its surface. The molecule must then penetrate the viable epidermis, the papillary dermis, and then the capillary walls and into systemic circulation. Along the way each of the above mentioned tissues will exhibit a different resistance to penetration by the same molecule; however, it is the stratum corneum that presents the greatest barrier to absorption of topical compositions or transdermally administered drugs. The stratum corneum, the outer horny layer of the skin, is a complex structure of compact keratinized cell remnants separated by lipid domains. Compared to the oral or gastric mucosa, the stratum corneum is much less permeable to outside molecules.

The flux of a drug across the skin can be increased by changing either a) the resistance (the diffusion coefficient), or b) the driving force (the solubility of the drug in the stratum corneum and consequently the gradient for diffusion). Many enhancer compositions have been developed to change one or more of these factors, and are known in the art. U.S. Patents Numbers 4,006,218, 3,551,154 and 3,472,931, for example, respectively describe the use of dimethylsulfoxide (DMSO), dimethyl formamide (DMF) and N,N-dimethylacetamide (DMA) to enhance the absorption of topically applied drugs through the stratum corneum. Combinations of enhancers consisting of diethylene glycol monoethyl or monomethyl ether with propylene glycol monolaurate and methyl laurate are disclosed in U.S. Patent 4,973,468 as enhancing the transdermal delivery of steroids such as progestogens and estrogens. A dual enhancer consisting of glycerol monolaurate and ethanol for the transdermal delivery of drugs is shown in U.S. Patent 4,820,720. U.S. Patent 5,006,342 lists numerous enhancers for transdermal drug administration consisting of fatty acid esters or fatty alcohol ethers of C₂ to C₄ alkanediols, where each fatty acid/alcohol portion of the ester/ether is of about 8 to 22 carbon atoms.

Even if a drug is amenable to transdermal delivery various problems may arise during the course of delivery. One such problem is known as the "burst effect". When a transdermal/transmucosal delivery system is applied to skin, it may deliver a very high dose of the drug initially which then drops or levels off after a period of time to reach acceptable plasma levels. The burst effect is caused by various factors. One such cause is the transdermal system itself. When a transdermal delivery system is applied to a biological membrane initially there exists a very high potential driving force within the system. As time passes the system loses drug and or enhancer, thus the potential driving force is also reduced resulting in less drug being delivered.

The invention herein described provides a solution to this problem, by virtue of the discovery that glycerin, when added to a transdermal formulation, effectively reduces the initial dose of a drug and thus reduces or eliminates the initial "burst effect" while maintaining a more uniform drug penetration over the intended duration of application.

Glycerin is well known in the industry as an emollient, such as taught by U.S. Pat. No. 4,687,481, and as an anti-irritant, as taught by U.S. Pat. No. 4,855,294. However, the applicants are unaware of any teaching in the art to the effect that glycerin functions to inhibit or reduce the initial rapid dose, i.e. burst effect, of a pharmaceutical agent in the manner described herein. In fact it is taught in U.S. Patent 4,855,294 that, in certain instances, glycerin has no effect on time averaged flux rates of drug across the tissues of the skin.

EP-A-0 020 905 discloses a drug composition for transdermal administration in the form of a film, which composition comprises glycerin to regulate the rate of drug release from the dosage form.

Accordingly, it is an objective of the present invention to provide a method for reducing the initial burst effect associated with transdermal/transmucosal drug delivery systems while maintaining a more uniform drug penetration over the intended duration of the delivery system without significantly reducing the dose administered.

According to the present invention there is provided use of a delivery system for moderating and maintaining transdermal drug delivery to the derma over the duration of application to a situs, said system comprising:
(a) a drug composition containing up to 99.9% by volume of a percutaneously absorbable drug and from 0.1 to 50% by volume of glycerin to moderate and maintain the transdermal delivery of said drug, and
(b) means for maintaining said drug composition in a drug transferring relationship with said derma.

While the invention has been found to be effective with all types of drugs, it is recognized that all drugs might not respond to the presence of glycerin in the same manner. Therefore, the invention is directed generally to reducing the initial burst effect in transdermal delivery systems and maintaining a more uniform drug permeation over the intended duration of application, particularly when used in conjunction with drug permeation enhancers. The combination of glycerin in a delivery system is not limited to any particular format or composition. Delivery patches, in liquid reservoir or matrix forms, or simple application of a drug applied to the skin in free form as a cream, gel, or ointment are all within the scope of the invention. The only limitation is that the composition must be effective for its intended use.

### DETAILED DESCRIPTION

The following definitions will be useful in describing the invention and will eliminate the need for repetitive explanations.

"Penetration enhancement" or "permeation enhancement" as used herein relates to an increase in the permeability of a biological membrane (i.e. skin or mucosal) to a drug, so as to increase the rate at which the drug permeates through the membrane. The enhanced permeation effected though the use of such enhancers can be observed, for example, by measuring the rate of diffusion of the drug through animal or human skin using a diffusion cell apparatus. The diffusion cell is described by Merritt et al.. Diffusion Apparatus for Skin Penetration, *J*. of *Controlled Release*, 1 (1984) pp. 161-162.

By "transdermal" delivery, the applicant intends to include both transdermal (or "percutaneous") and transmucosal administration, i.e., delivery by passage of drug through the skin or mucosal tissue. Hence the terms "transdermal" and "transmucosal" are used interchangeably unless specifically stated otherwise. Likewise the terms "skin", "derma", "epidermis", "mucosa" and the like shall be used interchangeably unless specifically stated otherwise.

By "afflicted situs" is meant a localized area of pathology, discomfort, infection, inflammation or lesion, and the immediately surrounding area.

By "application situs" is meant a site suitable for topical application with or without the means of a mechanical sustained release device, patch or dressing, e.g., behind the ear, on the arm, back chest, stomach, leg, top of foot, etc.

"Burst effect" refers to the high dose of drug delivered after initial application of a transdermal/transmucosal delivery system or formulation, followed by lower doses at later time periods, leading to a highly non uniform drug delivery profile.

"Carriers" or "vehicles" as used herein refer to carrier materials suitable for transdermal drug administration, and include any such materials known in the art, i.e., any liquid gel, solvent, liquid diluent, adhesive, or the like, which is nontoxic and which does not interact with other components of the composition in a deleterious manner. Carriers, which also may function as solvents in some instances, are used to provide the compositions of the invention in their preferred form. Examples include, but not limited to, water, ethanol, propanol, isopropanol, mineral oil, silicone, polyethylene glycol, polypropylene glycol, liquid sugars, waxes, petroleum jelly and a variety of other oils and polymeric materials along with polyacrylate, silicone, natural and synthetic rubbers or other adhesives.

By the term "drug" is meant any chemical material or compound suitable for transdermal or transmucosal administration which includes a desired biological or pharmacological effect by topical application to the "affliction situs" or by systemic delivery from the "application situs" to a desired target area. Such substances include the broad classes of compounds normally delivered through body surfaces such as the skin. In general, this includes therapeutic agents in all of the major therapeutic areas including, but not limited to, anti-infectives such as antibiotics and antiviral agents, analgesics and analgesic combinations, anorexics, antidiarrheal, antihistamines, anti-inflammatory agents, antimigraine preparations, antimotion sickness agents, antinauseants, antineoplastic, antiparkinsonism drugs, antipruritic, antipsychotic, antipyretics, antispasmodics including gastrointestinal and urinary, anticholinergic, sympathomimetic, xanthine derivatives, cardiovascular preparations including calcium channel blockers, beta-blockers, antiarrythmics, antihypertensives, diuretics, vasodilator including general coronary, peripheral and cerebral, central nervous system stimulants including cough and cold preparations, decongestants, diagnostics, hormones, immunosupressives, muscle relaxants, parasympatholytic, parasympathomimetic, psychostimulants, sedatives and tranquilizers.

By "effective" amount of a drug is meant a nontoxic but sufficient amount of a compound to provide the desired local or systemic effect. An "effective" amount of permeation enhancer as used herein means an amount selected so as to provide the desired increase in membrane permeability and, correspondingly, the desired depth of penetration, rate of administration and amount of drug. By "effective" amount of glycerin is meant the amount found beneficial in a particular delivery system to achieve the desired moderating of the initial burst effect followed by the relatively uniform delivery of the drug from the system.

By "system", "drug delivery system", "transdermal delivery system" or the like is meant a unit dosage form of a drug composition, including carriers, enhancers, glycerin and other components, which are contained in or accompanied by means for maintaining the drug composition in a drug transferring relationship with the derma, i.e. the skin or mucosa. Such means may be either a patch or other device of determined physical form to be held against the skin for continuous drug administration to an application situs for systemic transport or formulated in free form to be applied directly to an afflicted situs as a cream, gel, ointment and the like. Preferably the means used will be a device such as a matrix or liquid reservoir form patch.

By "drug composition", "drug/enhancer composition" or any similar terminology is meant a formulated composition containing the drug to be transdermally delivered in combination with such "carriers" or "vehicles", penetration enhancers, excipients, or any other additives. Because the invention is applicable to the utilization of glycerin as a modulator and maintainer of drug delivery with or without the presence of a penetration enhancer, the term "drug composition" shall be deemed to be inclusive of formulations containing enhancers unless such use is specifically excluded.

In the preferred embodiment of the present invention, glycerin is used to reduce the initial burst effect of a transdermally delivered drug and to also maintain a more uniform rate of drug penetration over the duration of the application of the transdermal system. The method involves treating the skin with glycerin prior to or concurrently with the administration of a drug delivery composition. The glycerin may be applied just prior to the administration of a drug composition. Alternatively, in the preferred embodiment, the drug composition that is to be transdermally administered is formulated so as to contain an effective amount of glycerin. As stated above, the drug composition may, in addition, include one or more carriers, vehicles, or excipients, and various agents and ingredients commonly employed in dermatological and cosmetic ointments or other preparations. Examples are, but not limited to, fragrances, pacifiers, preservatives, anti-oxidants, gelling agents, emollients, oils, stabilizers, coloring agents and the like may be present.

In the preferred embodiments, i.e., in which a drug composition for transdermal administration is formulated to contain an effective amount of glycerin with or without the presence of a penetration enhancer, the proportions of various drug and, when used, enhancer components are those typically in use. In other words, specific amounts of drug and/or enhancer are not critical and do not form part of the invention. The amount of glycerin making up an "effective amount" is normally in the range of 1.0% to 50% on a by volume basis, depending upon the type of formulation in which glycerin is to be incorporated, e.g. with or without the presence of an enhancer, incorporated into a matrix adhesive or placed in a liquid reservoir, used with or without a gelling agent, etc.

The maximum glycerin concentration will primarily be dictated by its functionality. For example, when formulating the drug, glycerin, an enhancer (if used) and any other component into a matrix/adhesive device, it is difficult to have a high glycerin concentration and still retain the adhesive qualities of the matrix. On the other hand, when used in liquid reservoir types of devices, too high a glycerin concentration may cause phase separation of various components or may interfere with a gelling agent such that the gelled formulation or ointment becomes stringy in texture and difficult to manufacture. For these reasons the total concentration of glycerin in a drug composition will not exceed about 50% by volume. In cases of formulation into a matrix/adhesive device the glycerin content will preferably not exceed about 20% by volume of the drug composition/adhesive combination. Most preferably the matrix/adhesive formulation will contain between about 0.5 and 10% by volume glycerin. The minimum glycerin concentration will also be dictated by functionality. There must be sufficient glycerin present to prevent the burst effect referred to above and to maintain transdermal drug on a somewhat consistent basis. In some instances amounts of a little as 0.1% by volume may be sufficient. When combined in a liquid reservoir type device the glycerin content has greater flexibility then in a matrix patch. Glycerin contents in liquid reservoir devices may preferably vary between about 1 and 50% by volume.

It will be appreciated by those skilled in the art that relative amounts of the other components in these compositions can vary greatly. For example, the amount of drug present in a given composition will depend upon a variety of factors, including but not limited to, the disease or condition to be treated, the nature of the drug, the activity of the drug, the desired effect, possible adverse reactions, the cost and availability of the drug, solubility of the drug, and other factors within the particular knowledge of the patient and physician. The amount of enhancer present in the composition will also depend on a number of factors, e.g., on the depth of cutaneous penetration desired, solubility of the drug in the enhancer, the strength of the particular enhancer, and the like.

While the invention is directed to the use of glycerin in any compatible drug composition, it is preferable that glycerin be formulated in a composition which does contain an enhancer. Although the invention is suitable for use with all enhancer compositions, there are certain enhancer compositions which are preferred. These will generally be one or more members selected from the group consisting of organic solvents and cell-envelope disordering compounds and mixtures thereof. Water may also be present. Preferred cell-envelope disordering compounds are members selected from the group consisting of isopropyl myristate, methyl laurate, oleic acid, oleyl alcohol, glycerol monoleate, glycerol dioleate, glycerol trioleate, glycerol monostearate, glycerol monolaurate, propylene glycol monolaurate and sorbitan mono-, di- and tri-esters and mixtures thereof. Preferred solvents are members selected from the group consisting of a C₂ or C₃ alcohol, and C₃ or C₄ diol, DMSO, DMA, DMF, 1-n-dodecyl-cyclazacyclo-heptan-2-one, N-methyl pyrrolidone, N-(2-hydroxyethyl) pyrrolidone, triacetin, propylene carbonate and dimethyl isosorbide and mixtures thereof. Ethanol, propanol, and isopropanol are particularly preferred as solvents. Water, as desired, may be added to either the cell envelope disordering compounds, solvents or mixtures thereof. In certain instances a compound can be both a cell envelope disordering compound and a solvent. Therefore, there can be a cross over from one category to the other without departing from the scope of this invention.

The method of application of the present invention may vary within limits, but necessarily involves applying the selected drug composition to the skin or other tissue where drug delivery is initiated at a moderated rate avoiding the usual "burst" effect and continues at a relatively sustained rate for a period of time sufficient to provide the desired pharmacological or biological response. When applied to an "afflicted situs" the method may involve a gel, lotion, cream, ointment, or the like. When applied to an "application situs" for systemic delivery to another location the method may involve the use of a drug delivery system device as taught, for example, in U.S. Patent Nos. 3,742,951, 3,797,494, 4,568,343 or 4,849,224. As noted above, glycerin is preferably co-administered with the drug/enhancer composition, however, it may also be applied in a pre-system application of the transdermal system or incorporated into an adhesive.

Preferred drug delivery devices include two types. The first is a matrix formulation where glycerin is incorporated into an adhesive layer.

The second type is a liquid reservoir device wherein the reservoir contains a combination of the drug to be delivered and glycerin. The liquid reservoir may or may not contain an enhancer or enhancers and other ingredients as defined above. The reservoir may be in a liquid form, or the liquid may be gelled or thickened by an agent such as mineral oil, petroleum jelly and various aqueous gelling agents and hydrophilic polymers. The above liquid reservoir or matrix device is brought in contact with the skin at the application situs and is held in place on the skin at the application situs by a suitable adhesive. The drug enhancer composition is applied to the skin through a permeable membrane forming the reservoir floor which is in direct contact with the skin.

The utilization of glycerin, as delineated herein, to moderate transdermal drug delivery is not characteristic of the stated prior art purposes for adding glycerin to formulations for dermal application. This invention is therefore novel and produces results which are unexpected from prior art statements relative to glycerin use.

### Experimental Skin Flux Studies

The in vitro human cadaver skin flux studies were conducted using modified Franz non-jacketed permeation cells. The temperature of the cells were maintained at 32°C by placing the cells in a water bath positioned over a stirring module. The epidermal membrane hid been separated from the human cadaver whole skin by the heat-separation method of Klingman and Christopher (*Arch. Dermatol*. 88:702 (1963)) involving the exposure of the full thickness skin to 60°C heat for 60 seconds, after which time the stratum corneum and part of the epidermis (epidermal membrane) was gently peeled off the dermis. The epidermal membrane was cut and placed between two halves of the permeation cell with the stratum corneum facing the donor compartment. The diffusion cells were allowed to hydrate at 32°C overnight with 0.02% (w/v) sodium azide solution in the receiver compartment. The next morning test formulations were placed on the stratum corneum. An appropriate receiver solution was placed in the receiver compartment in contact with the dermal side of the epidermis, to ensure sink conditions for the drug. At predetermined sampling intervals, the contents of the receiver compartment were sampled and analyzed by HPLC to determine the drug concentration.

Interval flux (µg/cm²/hr) was calculated based upon the incremental change in the cumulative amount delivered (µg/cm²) over a specified time period (hours).

### Example 1

Clonidine is a centrally acting alpha agonist and is an antihypertensive agent. It is an imidazoline derivative whose chemical name is 2,6-dichloro-N-2-imidazolidinylidenebenzenamine and is commercially available under the tradename CATAPRESS™. CATAPRESS™ systems were tested as described above, along with two inert adhesive matrix systems. One matrix used a solvent based polyacrylate adhesive , manufactured and marketed by Monosanto Chemical Company of St. Louis, Missouri under the tradename Gelva 737™ (herein referred to as G737), and the other a solvent based silicone adhesive manufactured and marketed by Dow Corning Corp. of Midland, Michigan under the tradename Bio-PSA X7-4302™ (herein referred to as X7-4302).

The matrix formulations were prepared as follows: a known weight of G737 or X7-4302 adhesive solution was weighed into a scintillation vial. Predetermined amounts of drug and/or glycerin were incorporated into the solution to give the required final dried film composition shown in Table 1. The scintillation vial was then tightly capped, sealed with parafilm, and rotated for a few hours until all ingredients were dissolved. Approximately 4 ml of this solution was then dispensed on a release liner and cast with a 10 mil gap casting knife. The cast was then dried in a convection oven at 70°C for 15 minutes to evaporate the solvent and to yield a dried film approximately 25.4 µm (2.0 mil) thick. A backing film was then laminated onto the dried adhesive film using a rubber roller. This matrix laminate was used to conduct the skin flux studies. The receiver compartment was filled with 0.067M NaH₂PO₄ solution containing 0.02% NaN₃.

The Results of the skin flux experiments are presented in Table 1. The CATAPRESS™ patch shows a high burst effect in the first 12 hour period. The matrix systems without glycerin also show a burst effect, but the systems with glycerin show a lower flux rate in the initial interval followed by a more constant permeation of the drug over 48 hours. The cumulative amount of clonidine delivered over 48 hours by all systems are substantially the same.

**Table 1**

| Drug | Adhesive Matrix Composition (% w/w) | | | Interval Flux (µg/cm²/h) | | | | | | Cumulative Permeation (µg/cm²/48h) |
|---|---|---|---|---|---|---|---|---|---|---|
| | Adhesive | Drug | Glycerin | 0-6 hr. | 6-13 hr. | 12-34 hr. | 24-30 hr. | 30-36 hr. | 36-48 hr. | |
| CATAPRESS | | | | 4.0 | 6.7 | 4.1 | 3.6 | 3.3 | 3.0 | 191 |
| Clonidine (G737) | 97 | 3 | 0 | 3.6 | 7.7 | 5.3 | 4.3 | 3.1 | 2.5 | 206 |
| Clonidine (G737) | 94.5 | 3 | 2.5 | 1.2 | 4.9 | 4.8 | 4.4 | 3.6 | 2.6 | 173 |
| Clonidine (X7-4302) | 98.25 | 1.75 | 0 | 3.7 | 8.8 | 6.7 | 4.9 | 3.6 | 2.8 | 240 |
| Clonidine (X7-4302) | 95.75 | 1.75 | 2.5 | 1.0 | 5.8 | 6.5 | 6.1 | 4.8 | 3.5 | 228 |

The above results are illustrative of formulations in matrix form which do not contain an enhancer. The non-glycerin containing formulations clearly demonstrate a "burst" effect whereas, the addition of a minor amount of glycerin, just as clearly moderates the burst effect and maintains the delivery rate of clonidine at a more constant level than when glycerin is not present.

### EXAMPLE 2

Progesterone is a naturally occurring female steroid hormone. Its molecular weight is 314.4.

Progesterone compositions were formulated using ethanol, water, glycerol monooleate (GMO), and methyl laurate (ML) as an enhancer with all but the base formulation containing glycerin in varying amounts as indicated by Table 2. Formulations were prepared using the second composition from Table 2 as exemplary. First, 0.15 grams of progesterone were added to a mixed solution made up of 2.8 ml H₂O, 1.0 ml glycerin, 0.1 ml GMO, 0.1 ml methyl laurate, and 6.0 ml EtOH. This drug solution mixture was subjected to sonication until the drug was dissolved. The next step was to add 0.15 g of Carbopol 1342 to the above solution followed by the addition of 66 ul 2N NaOH. The resulting mixture was homogenized using a virtishear homogenizer. The only variable to this method in preparing the other formulations shown in Table 2 was in the quantities of water and glycerin used.

Interval flux were evaluated as indicated above, and the results are set forth in Table 2. The receiver solution used in the receptor side of the cell was 3% bovine albumin with 0.01% gentamicin sulfate.

The interval flux in the initial interval (0-3h) is lower for the glycerin containing formulations than the non glycerin formulation. The extent of flux lowering in the initial interval is again a function of the concentration of glycerin in the formulation. All the glycerin containing formulations give a higher flux in the 9 - 24h period than the non glycerin formulation so that the cumulative amount of drug delivered over the 24 hours is somewhat similar for all the formulations.

**TABLE 2**

| Drug | Enhancer Composition (% v/v) | | | | | Interval Flux (µg/cm²/b) | | | | | Cumulative Permeation (µg/cm²/24h) |
|---|---|---|---|---|---|---|---|---|---|---|---|
| | EtCH | H²O | GMO | ML | Glycerin | 0-3 hr. | 3-6 hr. | 6-9 hr. | 9-12 hr. | 12-24 hr. | |
| Progesterone | 60 | 38 | 1 | 1 | 0 | 20.6 | 21.2 | 14.2 | 11.1 | 7.5 | 278 |
| Progesterone | 60 | 28 | 1 | 1 | 10 | 13.4 | 25.2 | 19.9 | 12.9 | 10.8 | 370 |
| Progesterone | 60 | 18 | 1 | 1 | 20 | 8.1 | 24.5 | 24.3 | 14.8 | 10.0 | 300 |

### EXAMPLE 3

Testosterone is a naturally occurring male steroid hormone which when not present causes hypogonadism. Native testosterone cannot be administered orally due to extensive pre-systemic metabolism. At present, the most effective and safest approach for treating testosterone deficiency states is the administration of synthetic testosterone esters, e.g. testosterone enanthate and testosterone cypionate, in 200 mg doses given by deep intramuscular injection at two-week intervals. One skilled in the art will recognize that testosterone, administered transdermally, would be superior to prior art methods. When attempts are made to deliver testosterone by means of a transdermal delivery system, the drug exhibits a very high initial burst effect which would not be beneficial to any known form of treatment of a testosterone deficiency. When glycerin is added to testosterone formulations the initial burst effect is diminished and a more constant level of delivery is established over the duration of administration.

Testosterone compositions were formulated using ethanol, water, glycerol monoleate (GMO) and methyl laureate (ML) as enhancer systems with all formulations containing varying amounts of glycerin as indicated by Table 3.

Formulations were prepared using the second composition from Table 3 as exemplary. First, 0.10 grams of testosterone was added to a mixed solution containing 1.5 ml H₂O, 3.0 ml glycerin, 0.25 ml GMO, 0.1 ml methyl laurate, and 5.0 ml EtOH. This drug solution mixture was subjected to sonication until the drug was dissolved. The next step was to add 0.30 g of Carbopol 1342 to the above solution followed by the addition of 132 ul 2N NaOH. The resulting mixture was homogenized using a virtishear homogenizer. The only variable to this method in preparing the other

**TABLE 3**

| Drug | Enhancer Composition (% v/v) | | | | | Interval Flux (µg/cm²/b) | | | | | Cumulative Permeation (µg/cm²/24h) |
|---|---|---|---|---|---|---|---|---|---|---|---|
| | EtCH | H²O | GMO | ML | Glycerin | 0-3 hr. | 3-6 hr. | 6-9 hr. | 9-12 hr. | 12-24 hr. | |
| Testosterone | 50 | 30 | 2.5 | 2.5 | 15 | 22.2 | 26.1 | 19.9 | 13.1 | 10.2 | 367 |
| Testosterone | 50 | 15 | 2.5 | 2.5 | 30 | 18.0 | 26.7 | 19.0 | 17.1 | 12.7 | 397 |
| Testosterone | 50 | 0 | 2.5 | 2.5 | 45 | 10.4 | 26.8 | 20.9 | 16.9 | 14.9 | 404 |

formulations shown in Table 3 was in the quantities of water and glycerin used.

Flux indices were evaluated as described above, with the receiver solution in the receptor side of the cell containing 3% bovine albumin and 0.01% gentamicin sulfate, and the results are set forth in Table 3.

Table 3 demonstrates the effect of increasing the concentration of glycerin on the flux rate of testosterone over a 24 hour period. The addition of increasing amounts of glycerin progressively lowers the flux in the initial interval (0-3 hrs), but the higher glycerin formulations give higher flux into the last interval (12-24 hrs) so that all the formulations are equivalent with respect to the cumulative amount of drug delivered over 24 hours.

The above examples are but illustrative of drugs or transdermal formulations which may be employed in operation of the present invention. The invention is directed to the discovery that the addition and/or utilization of glycerin moderates the "burst" effect associated with transdermal delivery and also maintains the transdermal drug delivery at a more constant rate than is experienced when glycerin is not present. Therefore, specific drugs and/or formulations are not critical as long as they are compatible with glycerin. Within the guidelines presented herein, a certain amount of experimentation to obtain optimal formulations can be readily carried out by those skilled in the art. Therefore, the invention is limited in scope only by the following claims and functional equivalents thereof.

## Claims

1. Use of a delivery system for moderating and maintaining transdermal drug delivery to the derma over the duration of application to a situs, said system comprising:
(a) a drug composition containing up to 99.9% by volume of a percutaneously absorbable drug and from 0.1 to 50% by volume of glycerin to moderate and maintain the transdermal delivery of said drug, and
(b) means for maintaining said drug composition in a drug transferring relationship with said derma.

2. Use as claimed in claim 1, characterised in that said drug composition and means for maintaining said drug composition are combined into a single formulation.

3. Use as claimed in claim 1 or 2, characterised in that said situs is an afflicted situs and the formulation is in free form as a member selected from the group consisting of a gel, lotion, cream and ointment.

4. Use as claimed in claim 1, 2 or 3, characterised in that said drug composition additionally contains a penetration enhancer.

5. Use as claimed in claim 4, characterised in that said penetration enhancer is a member selected from the group consisting of an organic solvent and a cell-envelope disordering compound and mixtures thereof.

6. Use as claimed in claim 5, characterised in that said organic solvent is a member selected from the group consisting of a C₂ or C₃ alcohol, and C₃ or C₄ diol, DMSO, DMA, DMF, 1-n-dodecyl-cyclazacyclo-heptan-2-one, N-methyl pyrrolidone and N-(2-hydroxyethyl) pyrrolidone, triacentin, propylene carbonate and dimethyl isosorbide and mixtures thereof and said cell-envelope disordering compound is a member selected from the group consisting of isopropyl myristate, methyl laurate, oleic acid, oleyl alcohol, glycerol monoleate, glycerol dioleate, glycerol trioleate, glycerol nonostearate, glycerol monolaurate, propylene glycol monolaurate and sorbitan esters and mixtures thereof.

7. Use as claimed in claim 6, characterised in that said organic solvent is a C₂ or C₃ alcohol.

8. Use as claimed in any one of claims 1, 2 and 4 to 7, characterised in that said situs is an application situs, said drug delivery system comprises a device of determined physical form, said means for maintaining said drug composition in a drug transferring relationship with the derma is an adhesive and said drug delivery system is affixed to a specified area of said derma by means of said adhesive for the duration of said application to said application situs.

9. Use as claimed in claim 8, characterised in that said device is a matrix/adhesive patch wherein said drug composition and said adhesive are combined.

10. Use as claimed in claim 9, characterised in that the glycerin content of said drug composition adhesive combination is from 0.1 to 20% by volume.

11. Use as claimed in claim 10, characterised in that the glycerin content of the drug composition adhesive combination is from 0.5 to 10% by volume.

12. Use as claimed in any preceding claim, characterised in that said drug is clonidine.

13. Use as claimed in claim 8, characterised in that said device is a liquid reservoir type patch and wherein said drug composition is contained in said reservoir.

14. Use as claimed in any preceding claim, characterised in that the drug is progesterone.

15. Use as claimed in any one of claims 1 to 13, characterised in that the drug is testosterone.

## Patentansprüche

1. Verwendung eines Abgabesystems zur Dämpfung und zum Aufrechterhalten einer transdermalen Arzneimittelverabreichung an die Haut über die Dauer der Anwendung auf eine Stelle, wobei das System folgendes umfaßt:
(a) eine Arzneimittelzusammensetzung, die bis zu 99,9 Volumen% eines perkutan absorbierbaren Arzneimittels und 0,1 bis 50 Volumen% Glycerin zur Dämpfung und zum Aufrechterhalten der transdermalen Abgabe des Arzneimittels enthält, und
(b) eine Vorrichtung zum Halten der Arzneimittelzusammensetzung in einer Arzneimittelübertragungsbeziehung mit der Haut.

2. Verwendung nach Anspruch 1, dadurch gekennzeichnet, daß die Arzneimittelzusammensetzung und die Vorrichtung zum Halten der Arzneimittelzusammensetzung zu einer einzigen Formulierung kombiniert sind.

3. Verwendung nach Anspruch 1 oder 2, dadurch gekennzeichnet, daß die Stelle eine kranke Stelle ist und die Formulierung in freier Form als Mitglied, das aus der aus einem Gel, einer Lotion, einer Creme und einer Salbe bestehenden Gruppe ausgewählt ist, vorliegt.

4. Verwendung nach Anspruch 1, 2 oder 3, dadurch gekennzeichnet, daß die Arzneimittelzusammensetzung zusätzlich einen Penetrationsverstärker enthält.

5. Verwendung nach Anspruch 4, dadurch gekennzeichnet, daß der Penetrationsverstärker ein Mitglied ist, das aus der Gruppe ausgewählt ist, die aus einem organischen Lösungsmittel und einer Zellenhülle-Störverbindung und Gemischen davon besteht.

6. Verwendung nach Anspruch 5, dadurch gekennzeichnet, daß das organische Lösungsmittel ein Mitglied ist, das aus der Gruppe ausgewählt ist, die aus einem C₂- oder C₃-Alkohol und C₃- oder C₄-Diol, DMSO, DMA, DMF, 1-n-Dodecyl-cyclazacyclo-heptan-2-on, N-Methylpyrrolidon und N-(2-Hydroxyethyl)pyrrolidon, Triacentin, Propylencarbonat und Dimethylisosorbid und Gemischen davon besteht, und die Zellenhülle-Störverbindung ein Mitglied ist, das aus der Gruppe ausgewählt ist, die aus Isopropylmyristat, Methyllaurat, Ölsäure, Oleylalkohol, Glycerolmonoleat, Glyceroldioleat, Glyceroltrioleat, Glycerolmonostearat, Glycerolmonolaurat, Propylenglykolmonolaurat und Sorbitanestern und Gemischen davon besteht.

7. Verwendung nach Anspruch 6, dadurch gekennzeichnet, daß das organische Lösungsmittel ein C₂- oder C₃-Alkohol ist.

8. Verwendung nach einem der Ansprüche 1, 2 und 4 bis 7, dadurch gekennzeichnet, daß der Ort ein Anwendungsort ist, wobei das Arzneimittelabgabesystem eine Vorrichtung mit bestimmter physikalischer Form umfaßt, wobei die Vorrichtung zum Halten der Arzneimittelzusammensetzung in einer Arzneimittelübertragungsbeziehung mit der Haut ein Haftmittel ist und das Arzneimittelabgabesystem auf einer festgelegten Fläche der Haut mittels des Haftmittels für die Dauer der Anwendung am Anwendungsort befestigt wird.

9. Verwendung nach Anspruch 8, dadurch gekennzeichnet, daß die Vorrichtung ein Matrix/Heftpflaster ist, wobei die Arzneimittelzusammensetzung und das Haftmittel kombiniert sind.

10. Verwendung nach Anspruch 9, dadurch gekennzeichnet, daß der Glyceringehalt der Kombination aus Arzneimittelzusammensetzung und Haftmittel 0,1 bis 20 Volumen% beträgt.

11. Verwendung nach Anspruch 10, dadurch gekennzeichnet, daß der Glyceringehalt der Kombination aus Arzneimittelzusammensetzung und Haftmittel 0,5 bis 10 Volumen% beträgt.

12. Verwendung nach einem vorangehenden Anspruch, dadurch gekennzeichnet, daß das Arzneimittel Clonidin ist.

13. Verwendung nach Anspruch 8, dadurch gekennzeichnet, daß die Vorrichtung ein Pflaster mit Flüssigkeitsreservoir ist, wobei die Arzneimittelzusammensetzung in dem Reservoir enthalten ist.

14. Verwendung nach einem vorangehenden Anspruch, dadurch gekennzeichnet, daß das Arzneimittel Progesteron ist.

15. Verwendung nach einem der Ansprüche 1 bis 13, dadurch gekennzeichnet, daß das Arzneimittel Testosteron ist.

## Revendications

1. Usage d'un système de délivrance pour modérer et conserver une délivrance transdermique d'un médicament à travers le derme pendant toute la durée d'application à un situs, ledit système comprenant :
(a) une composition médicamenteuse contenant jusqu' à 99,9 % en volume d'un médicament absorbable par voie percutanée et de 0,1 à 50 % en volume de glycérine pour modérer et conserver la délivrance transdermique dudit médicament, et
(b) des moyens pour conserver ladite composition médicamenteuse en relation de transfert médicamenteux avec ledit derme.

2. Usage selon la Revendication 1, ***caractérisé en ce que*** ladite composition médicamenteuse et les moyens pour conserver ladite composition médicamenteuse sont combinés en une formulation unique.

3. Usage selon la Revendication 1 ou 2, ***caractérisé en ce que*** ledit situs est un situs atteint et la formulation est sous forme libre en tant qu'un membre choisi dans le groupe constitué par un gel, une lotion, une crème ou un onguent.

4. Usage selon la Revendication 1, 2 ou 3, ***caractérisé en ce que*** ladite composition médicamenteuse contient additionnellement un stimulateur de pénétration.

5. Usage selon la Revendication 4, ***caractérisé en ce que*** ledit stimulateur de pénétration est un membre choisi dans le groupe constitué par un solvant organique et un composé désordonnant l'enveloppe cellulaire, et des mélanges de ceux-ci.

6. Usage selon la Revendication 5, ***caractérisé en ce que*** ledit solvant organique est un membre choisi dans le groupe constitué par un alcool C₂ ou C₃, et un diol C₃ ou C₄, le DMSO, la DMA (diméthylamine), le DMF, la 1-n-dodécyl-cyclo-aza-cyclo-heptan-2-one, la N-méthyl pyrrolidone et la N-(2-hydroxyéthyl) pyrrolidone, la triacétine, le carbonate de propylène et le diméthyl isosorbide, et le mélanges de ceux-ci, et ledit composé désordonnant l'enveloppe cellulaire est un membre choisi dans le groupe constitué par le myristate d'isopropyle, le laurate de méthyle, l' acide oléique, l'alcool oléique, le monooléate de glycérol, le dioléate de glycérol, le trioléate de glycérol, le monostéarate de glycérol, le monolaurate de glycérol, le monolaurate de propylène glycol et les esters de sorbitane, et les mélanges de ceux-ci.

7. Usage selon la Revendication 6, ***caractérisé en ce que*** ledit solvant organique est un alcool C₂ ou C₃.

8. Usage selon l'une quelconque des Revendications 1, 2, et 4 à 7, ***caractérisé en ce que*** ledit situs est un site d'application, ledit système de délivrance d'un médicament comprend un dispositif de forme physique déterminée, lesdits moyens pour conserver ladite composition médicamenteuse en relation de transfert médicamenteux avec le derme sont un adhésif, et ledit système de délivrance d'un médicament est fixé sur une zone spécifiée dudit derme au moyen dudit adhésif pour la durée de ladite application audit site d'application.

9. Usage selon la Revendication 8, ***caractérisé en ce que*** ledit dispositif est un dispositif transdermique ("patch") matrice/adhésif dans lequel ladite composition médicamenteuse et ledit adhésif sont combinés.

10. Usage selon la Revendication 9, ***caractérisé en ce que*** la teneur en glycérine de la combinaison composition médicamenteuse/adhésif est de 0,1 à 20 % en volume.

11. Usage selon la Revendication 10, ***caractérisé en ce que*** la teneur en glycérine de la combinaison composition médicamenteuse/adhésif est de 0,5 à 10 % en volume.

12. Usage selon l'une quelconque des Revendications précédentes, ***caractérisé en ce que*** ledit médicament est la clonidine.

13. Usage selon la Revendication 8, ***caractérisé en ce que*** ledit dispositif est un dispositif transdermique ("patch") du type à réservoir de liquide et dans lequel ladite composition médicamenteuse est contenue dans ledit réservoir.

14. Usage selon l'une quelconque des Revendications précédentes, ***caractérisé en ce que*** le médicament est la progestérone.

15. Usage selon l'une quelconque des Revendications 1 à 13, ***caractérisé en ce que*** le médicament est la testostérone.
